# FASCICULE DE BREVET EUROPEEN

(11) **EP 3 250 270 B1**
(45) Date de publication et mention de la délivrance du brevet: **24.04.2019**
(21) Numéro de dépôt: 16701360.6
(22) Date de dépôt: 25.01.2016
(51) Int. Cl.: A61M 5/32, A61M 5/31, A61M 5/00

(54) **DISPOSITIF DE PROTECTION D'UNE AIGUILLE, SERINGUE EQUIPEE D'UN TEL DISPOSITIF ET PROCEDE DE FABRICATION DE SERINGUES PRE-REMPLIES A AIGUILLE COLLEE**
VORRICHTUNG ZUM SCHUTZ EINER NADEL, SPRITZE MIT SOLCH EINER VORRICHTUNG UND VERFAHREN ZUR HERSTELLUNG VON FERTIGSPRITZEN MIT ZEMENTIERTER NADEL
DEVICE FOR PROTECTING A NEEDLE, SYRINGE PROVIDED WITH SUCH A DEVICE, AND METHOD FOR PRODUCING PRE-FILLED CEMENTED NEEDLE SYRINGES

(30) Priorité: 26.01.2015 FR 1550575; 02.06.2015 FR 1554990
(43) Date de publication de la demande: 06.12.2017
(73) Titulaire: Biocorp Production, 63500 Issoire (FR)
(72) Inventeur: ANEAS, Antoine, 63200 Menetrol (FR)
(74) Mandataire: Lavoix
(86) Numéro de dépôt international: PCT/EP2016/051408
(87) Numéro de publication internationale: WO 2016/120185

(56) Documents cités:
- EP-A1- 2 452 708
- WO-A1-03/068298
- WO-A1-2007/077463
- WO-A1-2010/019936
- WO-A1-2011/007194
- WO-A1-2011/110872
- WO-A1-2011/157930
- WO-A1-2013/084792
- WO-A1-2013/104736
- WO-A1-2013/134465
- WO-A1-2014/096825
- WO-A1-2014/131987
- WO-A1-2014/141470
- WO-A2-2016/115477
- GB-A- 2 114 006

## Description

L'invention concerne un dispositif de protection d'une aiguille, ainsi qu'une seringue à aiguille collée comprenant un tel dispositif.

De manière connue, un dispositif de protection d'une aiguille, qui peut être mieux connu sous le nom de « dispositif de sécurité après usage », a pour fonction de protéger l'aiguille d'une seringue en fin d'injection. Cela permet d'éviter de se blesser avec l'aiguille lorsque celle-ci est retirée du corps du patient et permet de lutter contre la transmission de maladies, comme le VIH.

Par exemple, GB-A-2 114 006 divulgue un pistolet injecteur pour animaux. Ce pistolet est destiné à un usage répété sur plusieurs animaux. Il comprend un manchon qui entoure une aiguille et qui comporte deux tubes télescopiques. Un ressort maintient le tube intérieur dans une position où il recouvre l'aiguille. Un capuchon permet de protéger l'aiguille. Ce capuchon comporte deux parties qui encapsulent un embout imprégné de liquide stérilisant et une partie détachable, qui est prévue pour être détachée du reste du capuchon par application d'un couple. Une fois la partie détachable retirée, le capuchon délimite une ouverture de passage de l'aiguille. Lors d'une injection, le pistolet est appuyé contre la peau de l'animal, ce qui a pour effet de rétracter le tube intérieur à l'intérieur du tube extérieur L'aiguille passe alors à travers l'embout stérilisé pour pénétrer dans la peau de l'animal. Lorsque le pistolet injecteur est retiré de l'animal, le tube intérieur revient en position de recouvrement de l'aiguille, laquelle est stérilisé au contact de l'embout. On réduit ainsi le risque de transmission de maladies d'un animal à l'autre. Le pistolet injecteur de GB-A-2 114 006 n'est pas destiné à un usage unique. Ainsi, après une injection, le tube intérieur peut être à nouveau rétracté à l'intérieur du tube extérieur pour découvrir l'aiguille. Ceci n'est pas compatible avec une seringue à usage unique, avec laquelle l'aiguille doit rester protégée après une injection.

Par ailleurs, WO-A-2013/134465, qui constitue l'art antérieur le plus proche, divulgue un dispositif de sécurité après usage pour seringue. Ce dispositif comprend un collier fixé autour du nez de la seringue. Un protège-aiguille souple est utilisé comme capuchon et a pour fonction de conserver l'aiguille propre, c'est-à-dire éviter la contamination du principe actif renfermé dans la seringue, et de protéger l'aiguille contre toute agression mécanique extérieure. Le dispositif comprend un manchon extérieur qui est mobile selon un axe longitudinal de la seringue à l'encontre d'un effort élastique généré par un ressort, entre une position avancée, où il recouvre l'aiguille, et une position reculée, où l'aiguille est découverte. Le collier comprend deux pions extérieurs, qui sont chacun engagés dans une glissière du manchon. Lors d'une piqûre, on retire le protège-aiguille souple et on recule manuellement le manchon dans une position intermédiaire, préliminaire à l'injection, dans laquelle une partie de l'aiguille dépasse à l'extrémité du manchon. Lorsque la seringue est amenée contre l'épiderme, l'aiguille pénètre dans l'épiderme et le manchon passe en position reculée. Lorsque l'injection est terminée et que l'aiguille est retirée du corps du patient, le manchon est rappelé en position avancée de manière à empêcher que l'aiguille soit découverte en fin d'injection et que que qu'un se pique. Un inconvénient de ce dispositif est que le manchon extérieur et le protège aiguille doivent être assemblés séparément sur la seringue car le protège-aiguille n'est pas intégré au dispositif de sécurité. Plus précisément, le protège-aiguille est assemblé chez le verrier, alors que le dispositif de sécurité est, le plus souvent, assemblé par le laboratoire pharmaceutique. Un autre inconvénient est que, lorsque le manchon est en position intermédiaire, l'aiguille est découverte et il y a un risque de piqure accidentelle.

A cet effet l'invention concerne un dispositif de protection d'une aiguille tel que défini à la revendication 1.

Grâce à l'invention, le protège-aiguille est directement intégré dans le dispositif de protection de l'aiguille, c'est-à-dire le dispositif de sécurité. Ce dispositif permet donc de remplir, outre sa fonction principale qui est de protéger l'aiguille en fin d'injection, les fonctions liées au protège-aiguille, c'est-à-dire empêcher la contamination du principe actif avant utilisation et protéger l'aiguille contre toute agression mécanique extérieure. De plus, le protège-aiguille et le manchon sont assemblés en même temps sur le corps de seringue, ce qui simplifie le procédé de fabrication de la seringue. Par ailleurs, du fait que l'aiguille est généralement taillée en biseau, des copeaux de matière peuvent être formés si l'embout dans lequel est enfoncée l'aiguille tourne autour de l'aiguille. Or, l'embout n'est pas solidaire en rotation de la gaine rigide. Ainsi, un couple mécanique peut être appliqué sur la gaine pour détacher les parties de la gaine l'une de l'autre, sans que l'embout ne soit entrainé en rotation. Le protège-aguille, du moins une partie de celui-ci, peut donc être désolidarisé du dispositif sans que des copeaux se forment à l'intérieur de l'aiguille.

Des aspects avantageux mais non obligatoires de l'invention sont définis aux revendications 2 à 5.

L'invention concerne également une seringue à aiguille collée selon la revendication 6.

Actuellement, les seringues pré-remplies avec aiguille collée sont fabriquées de la manière suivante : Le verrier fabriquant des corps de seringue en verre assemble un protège-aiguille souple ou rigide à l'extrémité de chaque corps de seringue avec aiguille collée, Les corps de seringue, alors équipés chacun d'un protège-aiguille, sont ensuite disposés dans un support en matière plastique, appelé « rack ». Ce rack est en fait une plaque délimitant une série de trous de réception des corps de seringue. Les racks sont ensuite disposés dans des containers de transport, qui sont operculés par un film en matériau synthétique. Les containers sont stérilisés à l'oxyde d'éthylène, qui est apte à traverser l'opercule. Lorsque les containers sont réceptionnés par le laboratoire pharmaceutique, ils sont ouverts dans une ambiance stérile et les racks sont sortis et placés sur des machines automatiques de remplissage. Chaque corps de seringue est alors rempli d'un principe actif, puis un piston, aussi appelé « joint de piston », est enfoncé à l'intérieur de chaque corps de seringue. Les corps de seringue sont alors sortis des racks pour les placer sur une ligne d'inspection et d'étiquetage. Une fois cette opération effectuée, les corps de seringue sont déplacés sur une autre ligne d'assemblage pour y incorporer le système de sécurité et une tige de poussée du piston, qui est apte à activer le système de sécurité une fois que l'injection est terminée.

L'inconvénient majeur de ce procédé est qu'il est nécessaire d'investir dans des infrastructures volumineuses et couteuses pour doter les seringues d'un système de sécurité après usage. Un autre inconvénient est que le système de sécurité choisi est particulièrement encombrant, de manière que les seringues ne peuvent être emballées ni avec un emballage primaire (blister) standard, ni avec un emballage secondaire (boite en carton) standard. En particulier, l'emballage final est particulièrement encombrant, ce qui entraine un coût de transport élevé. Enfin, la tige de poussée du piston utilisée n'est pas standard puisqu'elle comporte des rondelles pour activer le système de sécurité une fois l'injection terminée.

WO-A-20111110872 divulgue un procédé de fabrication de seringues pré-remplies à aiguille collée, dans lequel les corps de seringue sont disposés à l'intérieur de logements prévus dans un support. Chaque corps de seringue est équipé d'un protège-aiguille et d'un système de sécurité après usage. Le support des corps de seringue est placé dans un container, qui est fermé et stérilisé. Le support du container est ensuite sorti dans une ambiance stérile de manière à remplir chaque corps de seringue avec un principe actif. Le support utilisé est un support spécifique, dans lequel les logements sont conformés pur assurer un centrage des corps de seringue. L'inconvénient de ce procédé est donc que le support n'est pas un support standard, avec des trous de 9,3 mm de diamètre.

C'est à ces inconvénients qu'entend plus particulièrement remédier l'invention en proposant un procédé de fabrication de seringues pré-remplies qui peut être mis en oeuvre avec une installation moins volumineuse et moins couteuse.

A cet effet l'invention concerne aussi un procédé de fabrication tel que défini à la revendication 7.

Grâce à ce procédé, le dispositif de protection de l'aiguille peut être mis en place directement par le verrier, c'est-à-dire en même temps que le protège-aiguille, du fait que ce dernier est intégré au dispositif. Les laboratoires pharmaceutiques n'ont donc pas besoin de prévoir une ligne d'assemblage dédiée au montage du système de sécurité après remplissage. Cela permet de gagner de l'espace dans les infrastructures d'assemblage des seringues. En outre, le dispositif de protection intégrant le protège-aiguille est suffisamment compact, pour que les seringues peuvent être disposées, à l'étape b), avec leur dispositif de protection dans un support, ou « rack » standard. De même, des tiges de piston standard peuvent être utilisées. L'emballage final est aussi standard et peu encombrant. Ainsi, le procédé industriel de fabrication existant n'est pas perturbé, mais au contraire simplifié.

Des aspects avantageux mais non obligatoires de l'invention sont définis aux revendications 8 à 15.

L'invention et d'autres avantages de celle-ci apparaîtront plus clairement à la lumière de la description qui va suivre d'un mode de réalisation d'un dispositif de protection d'une aiguille et d'un procédé de fabrication conforme à leur principe, faite uniquement à titre d'exemple et en référence aux dessins annexés dans lesquels :
- les figures 1, 3, 5, 7, 9, 11 et 13 représentent chacune une vue de côté d'une seringue à aiguille collée, comprenant un dispositif de protection d'une aiguille conforme à l'invention,
- les figures 2, 4, 6, 8, 10, 12 et 14 représentent chacune une coupe longitudinale, à plus grande échelle, de la seringue correspondant aux figures mentionnées ci-dessus,
- les figures 15 et 16 sont des coupes longitudinales et à plus grande échelle, du dispositif de protection de la seringue des figures 1 à 14, dans deux plans de coupe différents,
- les figures 17 à 21 sont des coupes longitudinales représentant des étapes d'assemblage du dispositif des figures 1 à 16 sur une seringue à aiguille collée,
- la figure 22 est une vue en perspective représentant un container de transport pour la fabrication de seringues à aiguilles collées telle que représentée à la figure 1,
- la figure 23 est une coupe selon le plan XXIII de la figure 22,
- la figure 24 est une vue à plus grande échelle de l'encerclé XXIV de la figure 23, et
- les figures 25 à 27 sont des vues en perspective représentant chacune une étape du procédé de fabrication conforme à l'invention.

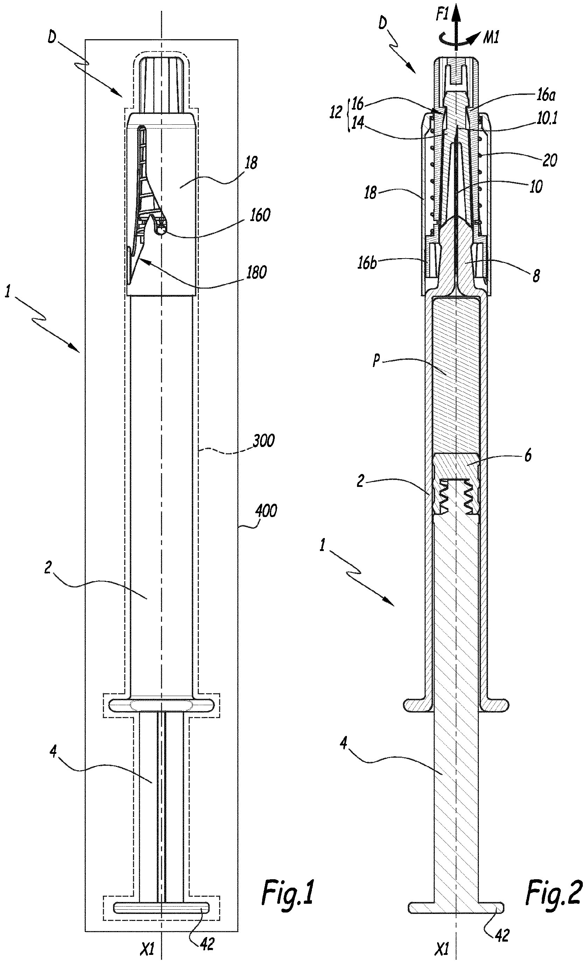

Sur chacune des figures 1 à 14 est représentée une seringue à aiguille collée 1. Cette seringue 1 est du type pré-remplie et s'étend selon un axe longitudinal X1. Elle comprend un corps de seringue 2, généralement en verre, qui est globalement tubulaire et centré sur l'axe X1. Le corps 2 comporte un nez 8 dans lequel est enfoncée une aiguille creuse 10. L'aiguille 10 est fixée à l'intérieur du nez 8 par collage. Le nez 8 présente un décrochement externe 80. L'aiguille 10 comporte une extrémité distale 10.1 taillée en biseau. Le corps de seringue 2 renferme un principe actif P, tel qu'un médicament. La seringue 1 comporte également une tige standard 4 qui est équipée à son extrémité d'un joint 6. Le joint 6 sert de piston pour éjecter le principe actif P à travers l'aiguille creuse 10, c'est pourquoi le joint 6 est couramment appelé « piston » ou « joint de piston ». Le joint 6 est attaché à la tige 4, c'est-à-dire qu'il est lié en translation avec la tige 4 dans les deux sens de déplacement. Plus précisément, la tige 4 est vissée à l'intérieur du joint 6. A l'opposé du joint 6 selon l'axe X1, la tige 4 est équipée d'une palette 42 sur laquelle l'utilisateur peut exercer un effort de poussée vers le nez 8. La tige 4 est déplaçable en translation par rapport au corps 2 selon l'axe X1, c'est-à-dire qu'elle est apte à coulisser à l'intérieur du corps de seringue 2.

Dans cette description, la direction avant ou distale désigne une direction parallèle à l'axe longitudinal X1 et tournée vers l'épiderme du patient dans des conditions normales d'utilisation de la seringue 1, alors que la direction arrière ou proximale est orientée à l'opposé par rapport à la zone d'injection, du côté de la palette 42.

La seringue 1 comporte, à l'avant, un dispositif D de protection de l'aiguille 10. Ce dispositif D est adapté pour être monté sur l'extrémité avant du corps de seringue. Le dispositif D intègre un protège-aiguille 12 qui permet, d'une part, de conserver l'aiguille 10 propre avant l'utilisation de la seringue 1 et éviter ainsi la pollution du principe actif P, et d'autre part, de protéger l'aiguille 10 contre toute action mécanique extérieure. Par exemple, le protège-aiguille 12 empêche l'aiguille 10 de se tordre ou de se briser avant usage.

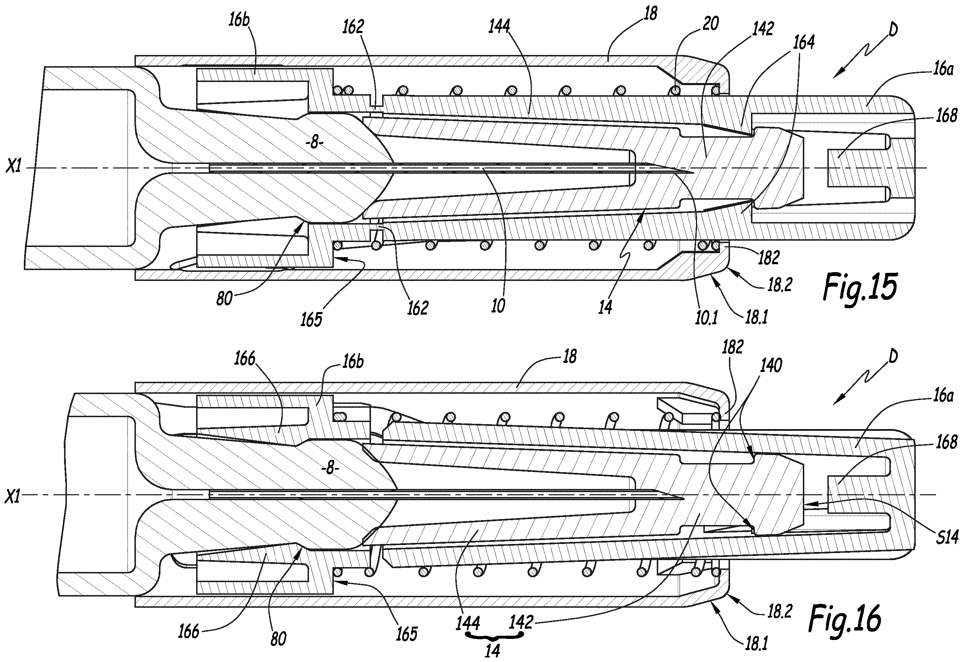

Le protège-aiguille 12 est un protège-aiguille rigide comprenant un embout souple 14 dans lequel est enfoncée l'aiguille 10 et une gaine rigide 16 qui enveloppe l'embout 14. L'embout 14 est en élastomère (caoutchouc) ou en matière thermoplastique injectable, alors que la gaine rigide 16 est en matière plastique, dans l'exemple en polyéthylène à haute densité (PEHD). Le protège-aiguille 12 est mieux visible aux figures 15 et 16. Comme visible sur ces figures, l'embout 14 comportes à l'avant, une section rétrécie 142 formant un épaulement annulaire 140 qui élargit le diamètre de l'embout 14 en allant vers l'avant. L'embout 14 comporte également, à l'arrière, une jupe 144 entourant une partie de l'aiguille 10. L'embout 14 est comprimé contre le nez 8 du corps de seringue 2. La jupe 144 est tronconique et s'élargit vers l'arrière de l'embout 14.

La gaine rigide 16 est en deux parties 16a et 16b qui sont détachables l'une de l'autre par un mouvement de rotation relatif entre les deux parties 16a et 16b. En effet, les deux parties de la gaine 16a et 16b sont reliées entre elles par des pontets sécables 162, conçus pour être rompus lors de l'application d'un moment M1 de rotation relative entre les deux parties de la gaine 16a et 16b. La partie 16a est disposée à l'avant de la partie 16b. Les parties 16a et 16b sont chacune de forme tubulaire centrée sur l'axe longitudinal X1.

La partie arrière 16b de la gaine 16 est encliquetée autour du nez 8 de la seringue 1, c'est-à-dire qu'elle comprend des moyens de fixation élastique autour du nez 8 de la seringue 1. Ces moyens de fixation comprennent des pattes élastiques 166 qui sont conformées pour venir se coincer dans le décrochement 80 du nez 8. La partie 16b est donc solidaire en rotation du corps de seringue 2. La partie arrière 16b de la gaine rigide 16 comporte aussi deux pions diamétralement opposés 160, qui font saillie radialement vers l'extérieur par rapport à l'axe longitudinal X1. Un seul de ces pions est cependant visible sur les figures.

Le dispositif D comprend des moyens de liaison en translation, le long de l'axe X1_{,} entre l'embout 14 et la partie 16a de la gaine 16. Ces moyens de liaison comprennent des dents 164 ménagées sur une surface radiale interne de la partie avant 16a de la gaine. Ces dents 164 font saillie radialement par rapport à l'axe longitudinal X1 vers l'intérieur de la gaine et coopèrent avec le rebord annulaire 140 de l'embout 14, de manière que l'embout 10 est lié en translation avec la partie avant 16a de la gaine 16. Cependant, une rotation de la gaine 16 autour de son axe n'entraine pas de rotation de l'embout 14, c'est-à-dire que la gaine 16 et l'embout 14 ne sont pas solidaires en rotation autour de l'axe longitudinal X1. La partie avant 16a de la gaine 16 comporte, à son extrémité avant, un ergot central 168 s'étendant axialement vers l'arrière. Il existe un jeu axial J2 d'environ 1 mm entre l'ergot 168 et une surface d'extrémité avant S14 de l'embout 14. Le jeu J2 est mesuré parallèlement à l'axe X1. Par ailleurs, il y a un jeu entre la surface extérieure de l'embout 14 et la surface intérieure de la gaine 16. Ce jeu est mesuré selon une direction normale à la surface extérieure de l'embout 14, qui est légèrement oblique par rapport à l'axe X1. Ainsi, l'embout 14 ne risque pas de tourner solidairement à la partie avant 16a de la gaine 16 par frottement.

Le dispositif de protection D comporte également un système de sécurité visant à protéger l'aiguille 10 après usage de la seringue 1, c'est-à-dire lorsque la seringue 1 est retirée du corps du patient. Ce système comprend un manchon extérieur 18, qui est disposé coaxialement autour de la gaine rigide 16. Le manchon 18 est fabriqué en matériau opaque, pour cacher complètement l'aiguille 10. Ce manchon 18 délimite un rebord radial interne 182 au niveau de son extrémité avant et deux évidements 180 dans lesquels les pions 160 sont respectivement insérés. Dans l'exemple, les pions 160 ne dépassent pas des évidements 160 vers l'extérieur. Les évidements 180 servent de guide aux pions 160. Chaque évidement 180 est globalement en forme de Y asymétrique, avec les branches du Y qui s'étendent vers l'arriére. Les branches du Y sont référencées 180a et 180c, alors que sa portion centrale est référencée 180b. Cette portion centrale 180b est une portion droite, c'est-à-dire un couloir. Le dispositif D comprend également des moyens de verrouillage du manchon 18 en position avancée, qui sont activés en fin d'injection. Dans l'exemple, ces moyens de verrouillage sont formés par un logement 180d qui s'étend, à partir de la branche 180c, vers l'avant.

Le manchon extérieur 18 est mobile axialement, c'est-à-dire le long de l'axe X1, entre une position avancée, où il est recouvre l'aiguille 10 et une position reculée, où l'aiguille 10 est découverte. Le système de sécurité comprend des moyens de rappel élastique du manchon extérieur 18 en position avancée. Ces moyens de rappel comprennent un ressort hélicoïdal 20 qui est intercalé entre le rebord radial interne 182 du manchon 18 et un épaulement 165 formé sur la partie arrière 16b de la gaine rigide 16. Le ressort hélicoïdal 20 présente un pas à droite, c'est-à-dire que le sens d'enroulement du ressort 20 est à droite. Cela signifie que le ressort 20 est enroulé à droite, ou dans le sens horaire, lorsque l'on regarde le ressort 20 par le bas aux figures 1 à 14, c'est-à-dire du côté arrière.

Il existe un jeu mécanique radial entre le manchon extérieur 18 et la gaine rigide 16, de sorte que le manchon 18 peut coulisser autour de la gaine 16 sans frottements. En revanche, Il n'existe pas ou peu de jeu mécanique radial entre le manchon 18 et la surface extérieure du corps de seringue 2, de sorte que le dispositif D n'est pas très encombrant radialement En particulier, l'épaisseur du manchon 18 est choisie pour que les seringues 1 puissent être insérées dans les trous d'un support standard.

Par ailleurs, le manchon 18 comporte à son extrémité distale un chanfrein annulaire 18.1 qui converge par rapport à un axe central du manchon 18 vers l'avant et qui est relié au rebord radial interne 182 par un congé périphérique 18.2.Ci-dessous sont décrites différentes étapes d'utilisation de la seringue 1 en référence aux figures 1 à 14.

Tout d'abord, l'utilisateur doit retirer le protège-aiguille rigide 12 pour pouvoir réaliser l'injection. Pour ce faire, il applique le moment M1 autour de l'axe X1, comme représenté à la figure 2, pour tourner la partie avant 16a par rapport à la partie arrière 16b et rompre les pontets 162. Une fois les pontets 162 rompus, l'utilisateur peut retirer la partie avant 16a de la gaine rigide 16, comme représenté par la flèche F1 à la figure 2. Le retrait de la partie 16a entraine solidairement le retrait de l'embout 14 par coopération des dents 164 avec le rebord annulaire 140 de l'embout 14. Ainsi, l'embout 14 et la partie avant 16a de la gaine 16 sont retirés de la seringue 1 sans faire tourner l'embout 14 autour de l'aiguille 10, de manière que l'extrémité distale 10.1 de l'aiguille 10, taillée en biseau, ne forme pas de copeaux de matière susceptibles de pénétrer dans l'aiguille 10.

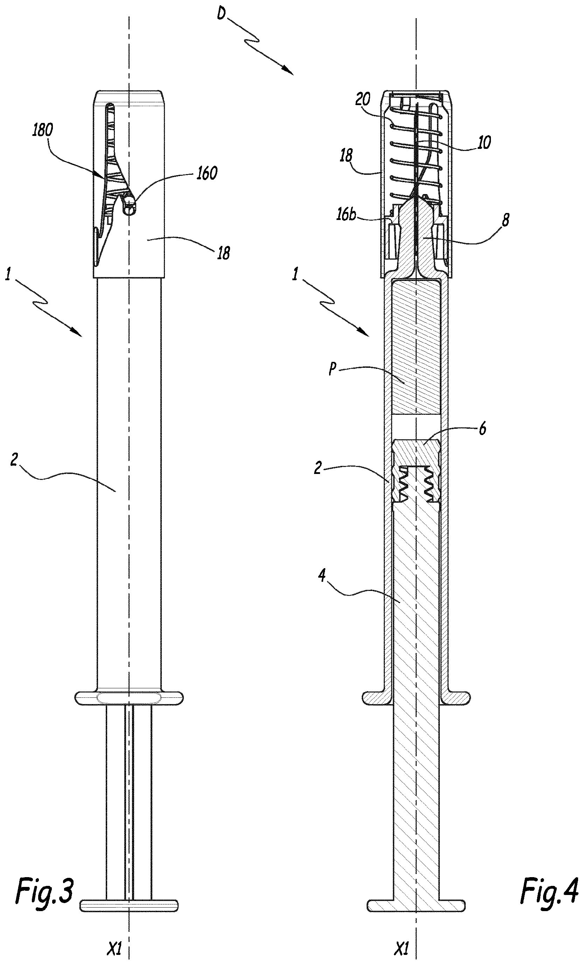

Le retrait de l'embout 14 et de la partie avant 16a de la gaine 16 amène la seringue 1 dans la configuration des figures 3 et 4. Dans cette configuration, l'aiguille 10 est entièrement recouverte par le manchon 18. Tant que la seringue 1 n'a pas été utilisée, les pions 160 de la partie arrière 16b de la gaine 16 sont logés dans la branche 180a des évidements 180.

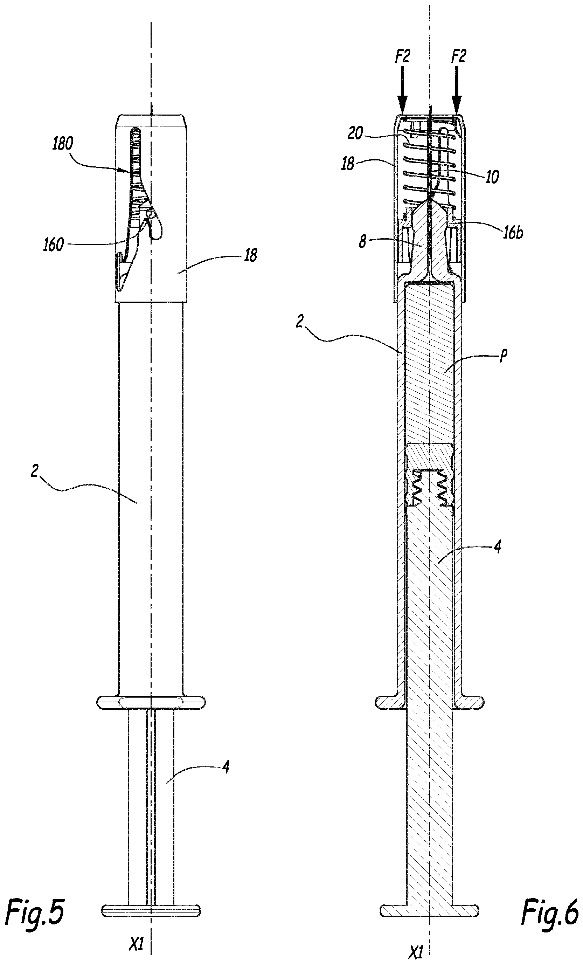

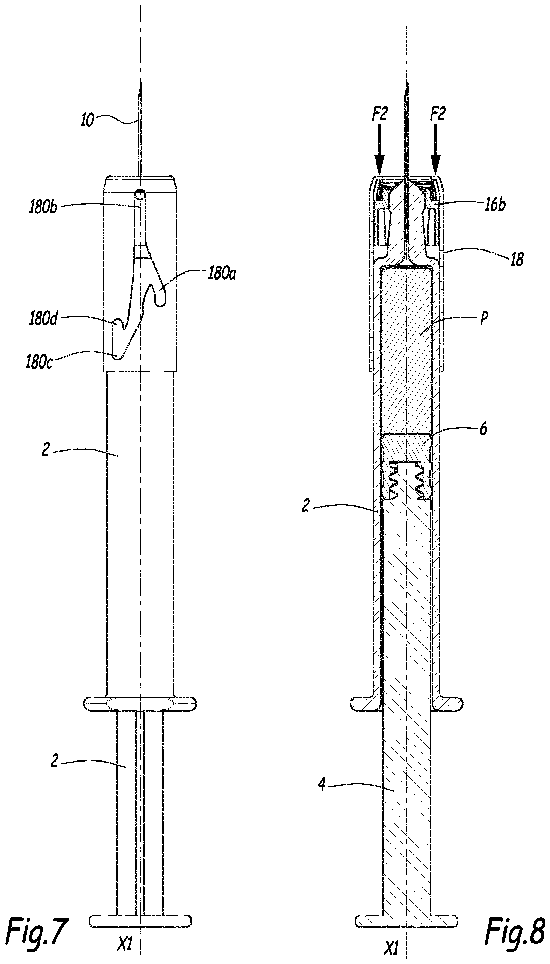

En référence aux figures 5 et 6, lorsque la seringue 1 est amenée contre l'épiderme d'un patient, la pression exercée par le manchon 18 sur la peau entraine le recul du manchon 18, comme représenté par les flèches F2 à la figure 6. Le ressort 20 est alors comprimé, l'aiguille 10 pénètre dans l'épiderme et les pions 160 se déplacent de la branche 180a jusque dans la portion centrale 180b. Le manchon 18 recule autour du corps de seringue 2. Ainsi, l'aiguille 10 n'est pas découverte tant que la seringue 1 n'est pas amenée contre l'épiderme du patient, à la différence des matériels selon WO-A-2013/134465 et WO-A-2007/077463, où l'aiguille est en partie découverte avant que la seringue ne soit amenée contre le corps du patient. Autrement dit, le manchon 18 n'est pas reculé préalablement à l'injection pour laisser apparaitre l'aiguille 10. Ainsi, il n'y a pas de risque de piqûre accidentelle avant l'injection. La poursuite du mouvement amène le manchon extérieur 18 vers sa position reculée, dans laquelle il ne recouvre plus l'aiguille 10. La poursuite du mouvement s'effectue jusqu'à ce que les pions 160 arrivent au fond du couloir 180b des évidements 180, comme représenté aux figures 7 et 8.

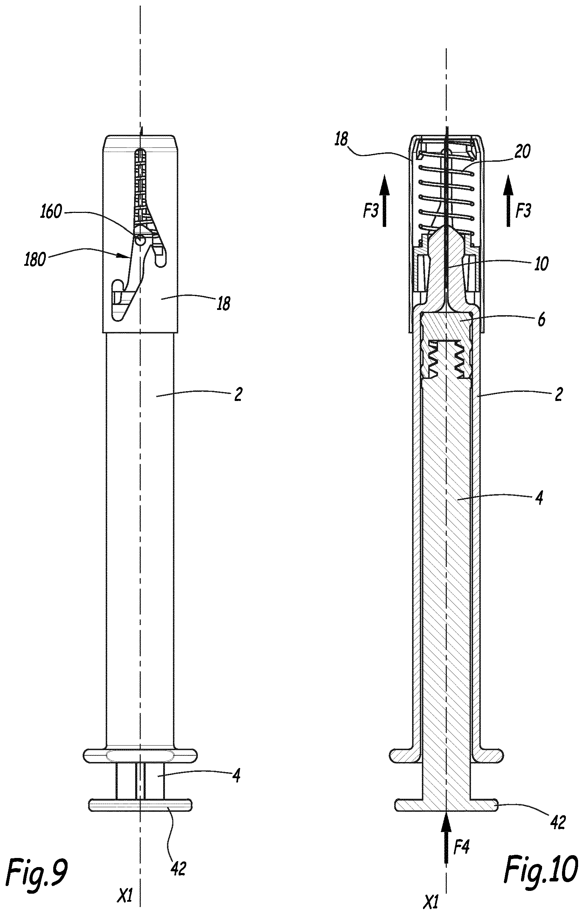

Dans la configuration des figures 7 et 8, l'aiguille 10 de la seringue 1 est complètement enfoncée dans l'épiderme du patient. L'utilisateur peut alors appuyer sur la palette 42 de la tige 4 pour éjecter le principe actif P contenu à l'intérieur de la seringue 1 dans le corps du patient, comme représenté par la flèche F4 à la figure 10.

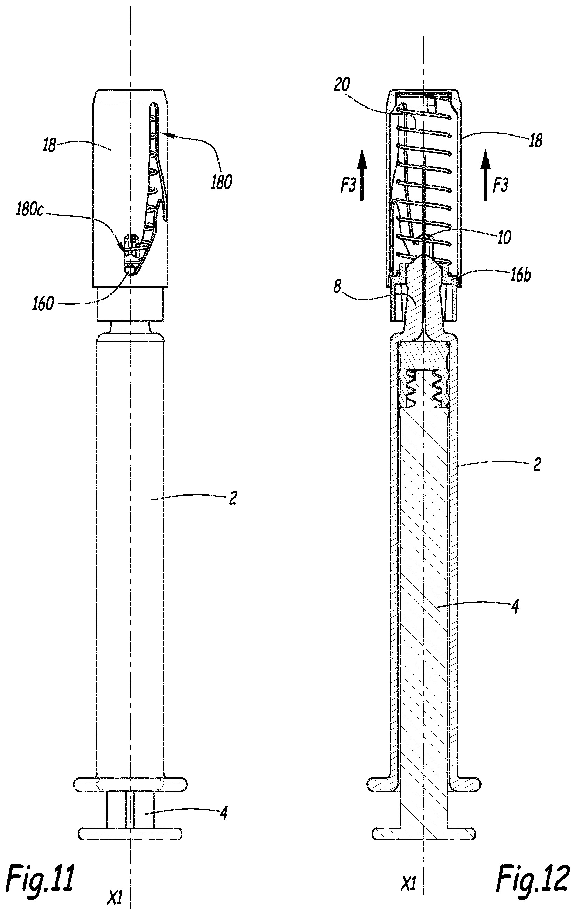

Lorsque l'utilisateur retire la seringue 1 du corps du patient, le manchon extérieur 18 est rappelé élastiquement en position avancée par le ressort 20, comme représenté par les flèches F3 à la figure 10. Le manchon extérieur 18 revient alors en recouvrement de l'aiguille 10 et les pions 160 coulissent dans le couloir 180b des évidements 180 en direction de la branche 180c, La seringue 1 se trouve alors dans la configuration des figures 11 et 12, qui correspond à une configuration de fin d'injection.

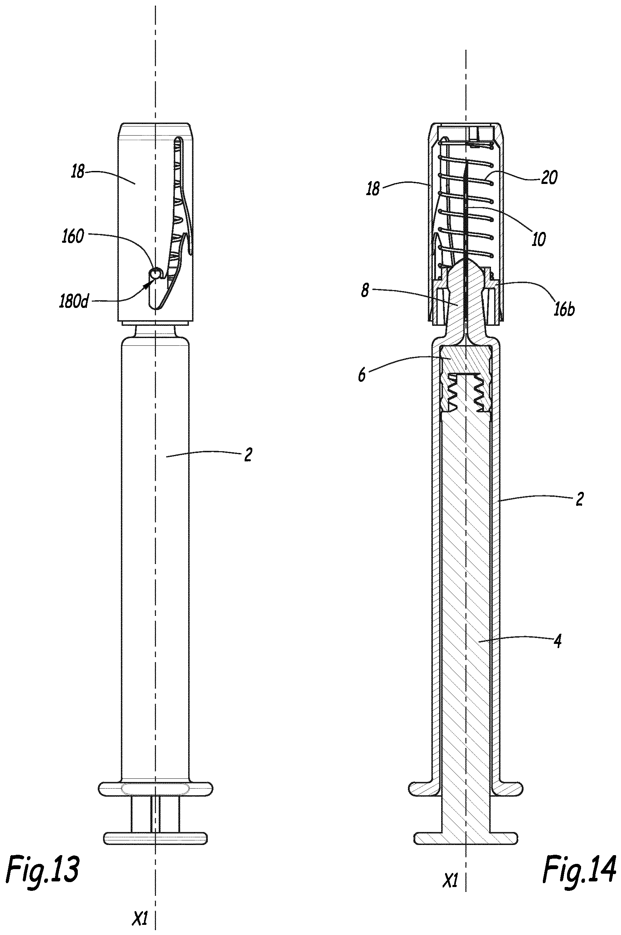

Si, après usage de la seringue 1, un utilisateur maladroit appuie sur le manchon 18, c'est-à-dire tente de reculer le manchon 18, les pions 160 se déplacent alors dans le logement 180d des évidements 180 et le déplacement du manchon 18 vers l'arrière est bloqué, comme représenté sur les figures 13 et 14. Cela constitue une sécurité supplémentaire puisque l'aiguille 10 ne peut plus être découverte en fin d'injection. Plus précisément, le déplacement des pions 160 de la branche 180c vers le logement 180d est favorisé du fait que le ressort 20 a un sens d'enroulement à droite. En effet, ce ressort 20 exerce, lorsqu'il est comprimé, un couple sur le manchon 18 qui est dirigé, du fait de son sens d'enroulement, dans le sens antihoraire en vue de dessus à la figure 11, c'est-à-dire lorsque l'on regarde la seringue 1 du côté de l'aiguille 10. Ce couple permet d'éviter que les pions 160 retournent en direction du couloir 160b si l'utilisateur tente de reculer le manchon 18 après l'injection. Ce couple permet également de guider correctement les pions 160 dans le couloir 180b jusqu'à la branche 180c des évidements 180.

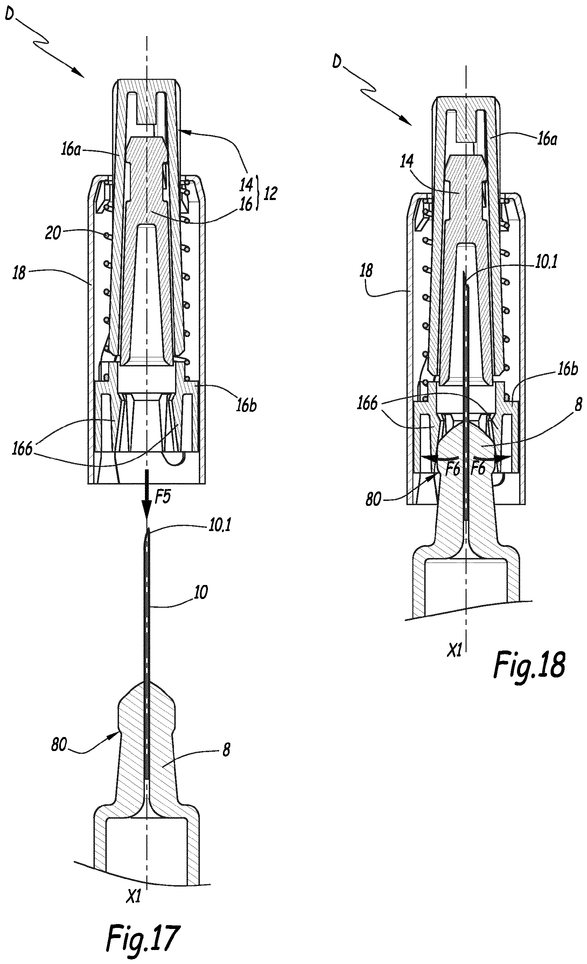

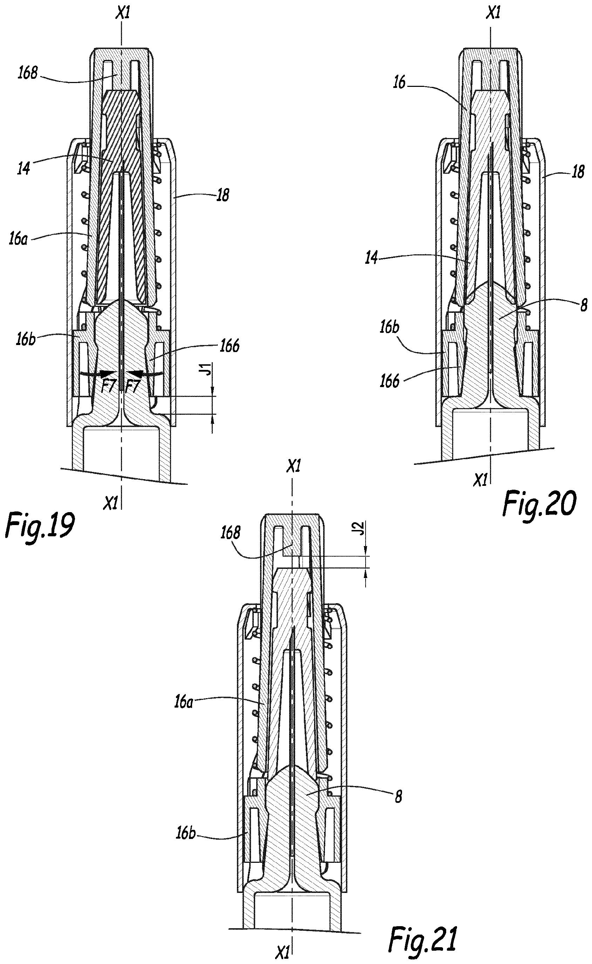

Sur les figures 17 à 21 sont représentées les étapes de montage du dispositif de protection D sur le nez 8 du corps de seringue 2. Une première étape du montage du dispositif de protection D consiste à rapprocher le dispositif D du nez 8, comme représenté par la flèche F5 à la figure 17. En poursuivant le mouvement d'approche dans le sens de la flèche F5 les pattes élastiques 166 sont alors déformées selon une direction radiale centrifuge F6 au contact du nez 8, comme visible à la figure 18. Une fois que les pattes 166 ont dépassé le décrochement 80 du nez 8, elles viennent s'encliqueter contre ce dernier par retour élastique de la matière, comme représenté par les flèches F7 à la figure 19. Le nez 8 de la seringue 1 est conformé pour bloquer le dégagement des pattes 166. Il existe un jeu J1 d'environ 1,5 mm entre l'extrémité libre des pattes 166 et le corps de seringue 2. Le jeu J1 est mesuré parallèlement à l'axe X1.

Lors d'une dernière étape illustrée par les figures 19 à 21, on appuie sur le protège-aiguiile 12 de manière à bien enfoncer l'aiguille 10 à l'intérieur de l'embout 14. Comme visible à la figure 20, l'embout 14 se retrouve alors comprimé axialement, entre l'ergot 168 de la gaine rigide 16 et le nez 8 du corps de seringue 2 et le jeu J1 entre l'extrémité libre des pattes 166 et le corps de seringue 2 est rattrapé. Lorsque l'on relâche la pression sur le protège-aiguille 12, la matière de l'embout 14 reprend sa forme initiale et repousse la gaine 16 vers l'avant. La gaine 16 revient alors en position d'encliquetage et un jeu axial J2 existe entre la surface d'extrémité avant S14 de l'embout 14 et l'ergot 168.

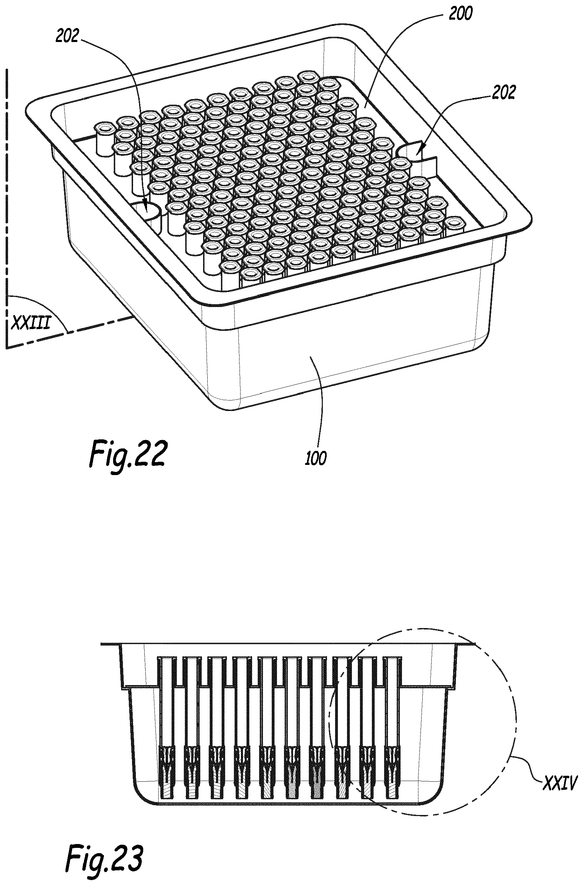

Ci-dessous est décrit, en référence aux figures 22 à 27, un procédé de fabrication de seringues pré-remplies à aiguille collée 1, telles que décrites précédemment. Dans le présent document, une pièce est considérée comme « standard » si elle se trouve déjà dans le commerce, c'est-à-dire si elle est déjà utilisée dans les procédés de fabrication existants.

Le procédé comprend une première étape a) consistant à monter un dispositif de protection D sur chaque seringue à aiguille collée 1. Cette première étape a) est effectuée par un verrier fabricant des corps de seringue 2. Comme le dispositif de protection D intègre le protège-aiguille 12, le système de sécurité et le protège-aiguille 12 sont montés d'un seul bloc sur le corps de seringue 2. Cela permet de simplifier le procédé d'assemblage existant. En outre, grâce aux moyens de fixation élastique, le montage du dispositif D sur un corps de seringue 2 s'effectue simplement par rapprochement des deux éléments. Une deuxième étape b) consiste à disposer les seringues 1, chacune équipée d'un dispositif de protection D, dans des logements 0204 prévus dans un support standard 200. Ce support 200 est couramment appelé « rack » et consiste en une plaque rectangulaire en matière plastique, qui est trouée. Les trous 0204 de la plaque 200 forment les logements de réception des seringues 1. Les trous 0204 ont un diamètre inférieur au diamètre d'une surface d'enveloppe extérieure du dispositif D. De manière standard, ces trous 0204 ont un diamètre de 9,3 mm. Une surface d'enveloppe extérieure du dispositif D présente un diamètre extérieur qui est donc inférieur à 9,3 mm, dans l'exemple de l'ordre de 9 mm. Les trous O204 sont délimités par des coionnettes 204 qui font saillie vers le haut perpendiculairement à la plaque 200. Le support 200 délimite seize rangées de dix trous O204.

Grâce à la présence du chanfrein 18.1 et du congé 18.2 prévus à son extrémité distale, chaque dispositif D est inséré sans accroc dans un logement O204 du support 200. Autrement dit, lorsque les seringues sont insérées dans le support 200, la surface extérieure du manchon 18 glisse contre la paroi du trou correspondant O204.

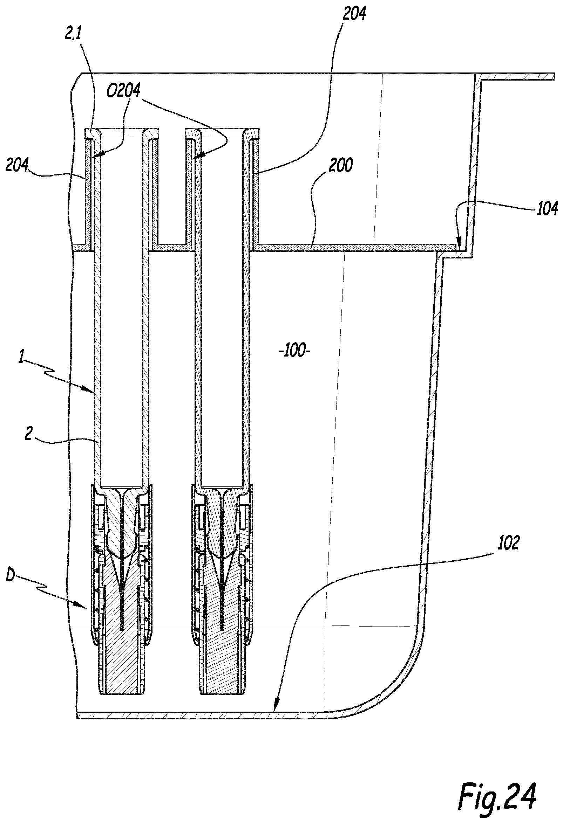

Le corps 2 de chaque seringue comporte, à une extrémité opposée au nez 8, un épaulement annulaire 2.1 prévu pour venir en appui contre l'extrémité libre d'une colonnette 204, de manière à empêcher le corps de seringue 2 de tomber sous l'effet de la gravité. La plaque 200 comporte deux poignées 202 facilitant sa préhension. Lorsque toutes les seringues sont disposées dans le rack 200, celui-ci est positionné, lors d'une troisième étape c), à l'intérieur d'un container standard 100. Le container 100 comprend à cet effet deux épaulements opposés 104, qui s'étendent chacun sur la totalité d'un côté du container 100. Comme visible aux figures 23 et 24, les seringues 1 ne touchent pas le fond 102 du container 100 lorsque le rack 200 est placé dans le container 100.

Le container 100 est ensuite operculé de manière étanche par un film en matériau synthétique non représenté, qui est non-étanche aux gaz. Le container 100 est rendu stérile par injection de gaz, notamment de l'oxyde d'éthylène. L'oxyde d'éthylène pénètre à travers l'opercule du container, ce qui permet de stériliser les seringues à l'intérieur du container. A l'issue de l'étape c), les containers 100 sont envoyés par le verrier au laboratoire pharmaceutique. Autrement dit, le procédé comprend une étape postérieure à l'étape c) consistant à transporter les containers 100 d'un point à un autre, en l'occurrence du verrier au laboratoire pharmaceutique.

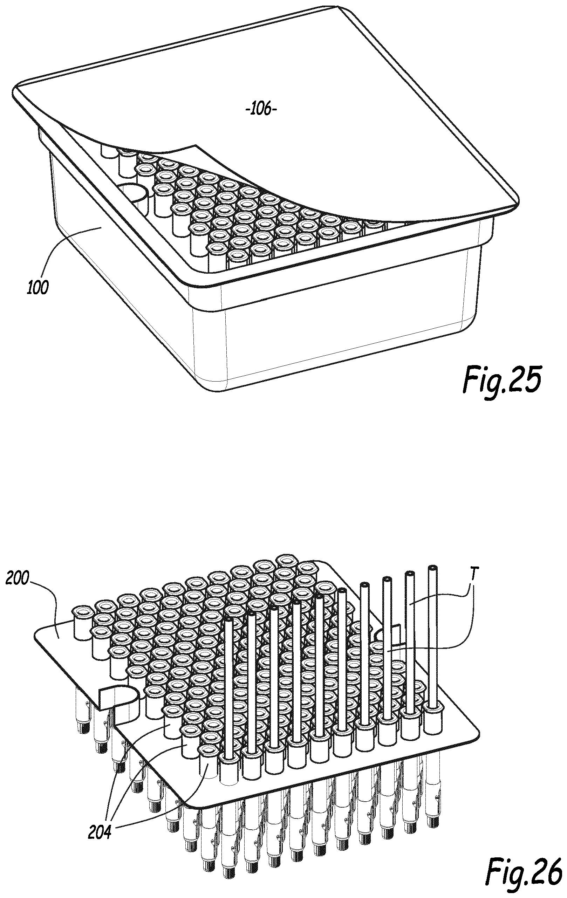

Comme visible à la figure 25, les containers 100 sont ouverts, lors d'une quatrième étape d), chez le laboratoire pharmaceutique dans une ambiance stérile et les racks 200 sont sortis de manière automatisée des containers 100. Lors d'une cinquième étape e), les racks 200 sont placés sur des machines automatiques de remplissage non-représentées pour remplir les corps de seringue 2 avec un principe actif P, comme un médicament. Comme visible à la figure 26, la machine de remplissage comprend une rangée de dix tubes de remplissage T, adaptés pour être inséré chacun dans un corps de seringue 2. Le principe actif est alors injecté à travers les tubes T. Une fois que tous les corps de seringue d'une rangée sont remplis, les tubes T sont sortis hors des corps 2 et le support 200 est déplacé pour remplir une autre rangée de seringues.

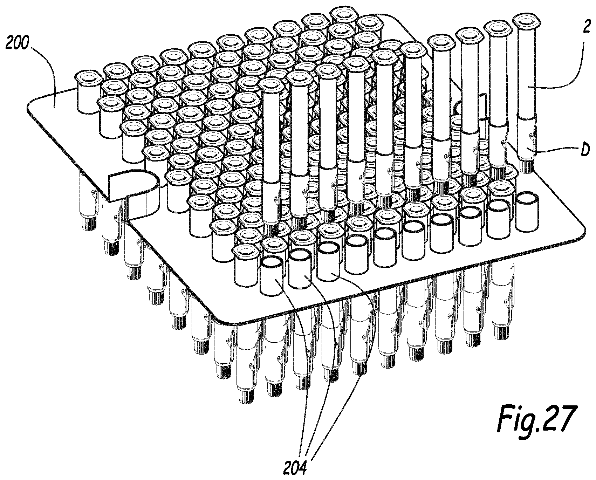

Une fois toutes les seringues 1 remplies, un piston, ou joint de piston 6 est inséré lors d'une sixième étape f) à l'intérieur de chaque corps de seringue 2. Comme visible à la figure 27, les corps 2 des seringues 1, alors remplis de principe actif, sont sortis, lors d'une septième étape g), des racks 200. L'extraction des seringues hors du support 200 s'effectue sans accrocs du fait de la présence du chanfrein annulaire 18.1 et du congé 18.2 sur chaque dispositif D. Autrement dit, lorsque les seringues sont extraites du support 200, la surface extérieure du manchon 18 glisse contre la paroi du trou correspondant O204. Les seringues 1 sont ensuite placées sur une ligne pour l'inspection, l'assemblage de la tige de piston 4 et l'étiquetage. Précisément, la tige 4 est vissée dans le joint 6.

Lors d'une huitième étape h), les seringues pré-remplies 1 sont emballées individuellement avec un emballage primaire 300 et un emballage secondaire 400. L'emballage primaire 300 est un emballage coque transparent standard, mieux connu sous le nom de « blister». L'emballage secondaire 400 est une boite en carton standard. Le produit emballé est compact et donc facilement transportable. Ces emballages sont représentés, respectivement en traits pointillés et en traits pleins à la figure 1 uniquement.

Grâce à ce nouveau procédé, les seringues 1 sont livrées au laboratoire pharmaceutique avec leur dispositif de protection D intégré, si bien que le laboratoire pharmaceutique n'a pas à assembler le dispositif de protection sur chaque seringue. Le laboratoire pharmaceutique n'a donc pas besoin de disposer d'une ligne d'assemblage dédiée au montage des dispositifs de protection sur les seringues, ce qui permet d'économiser de la place. De plus, le dispositif de protection D est très compact, de sorte que la seringue 1 peut être disposée dans un trou du rack directement avec le dispositif de protection.

En variante non représentée, des moyens de rappel différents d'un ressort peuvent être envisagés pour rappeler le manchon extérieur 18 en position avancée en fin d'injection.

En variante non représentée, un seul évidement 180 est pratiqué dans le manchon 18. De même, le manchon 18 peut délimiter un nombre d'évidements 180 strictement supérieur à deux, par exemple égal à trois.

En variante non représentée, les parties 16a et 16b de la gaine 16 peuvent être vissées l'une à l'autre ou liés par un mécanisme de verrouillage rotatif. Par exemple, le mécanisme peut comprendre un pion guidé dans une rainure curviligne ou coudée. Ce type de mécanisme est couramment appelée mécanisme de verrouillage à baïonnette. Dans tous les cas, les parties 16a et 16b sont détachables l'une de l'autre par un mouvement de rotation relatif entre les deux parties.

En variante non représentée, le joint de piston 6 n'est pas attaché à la tige 4, c'est-à-dire que la tige 4 est en appui simple contre le joint 6. Le joint 6 n'est alors lié à la tige 4 que dans un sens de déplacement.

Selon une autre variante non représentée, le container 100 délimite une ouverture ou une partie transparente pour la visualisation des seringues 1 de l'extérieur

Les caractéristiques des variantes et modes de réalisation envisagés ci-dessus peuvent être combinées entre elles pour générer de nouveaux modes de réalisation de l'invention.

## Revendications

1. Dispositif (D) de protection d'une aiguille (10), prévu pour être monté sur un nez (8) d'un corps (2) de seringue (1) à aiguille collée, ce dispositif comprenant :
- un manchon extérieur (18), qui est mobile le long d'un axe longitudinal (X1) entre une position avancée, où il recouvre l'aiguille, et une position reculée, où il ne recouvre pas l'aiguille,
- des moyens (20) de rappel du manchon en position avancée, et
- des moyens (180d) de verrouillage du manchon (18) en position avancée en fin d'injection.
**caractérisé en ce que**:
- le dispositif comprend en outre un protège-aiguille rigide (12) comprenant un embout souple (14) enveloppé dans une gaine rigide (16) cette gaine comprenant une première partie (16b) et une deuxième partie (16a), qui est détachable de la première partie par un mouvement de rotation par rapport à la première partie, la première partie (16b) de la gaine comprenant des moyens (166) de fixation élastique autour du nez (8) de la seringue, qui permettent de solidariser la première partie (16b) de la gaine et le corps de seringue (2) en rotation,
- les deux parties (16a, 16b) de la gaine sont reliées entre elles par des pontets sécables (162), conçus pour être rompus lors de l'application d'un moment (M1) de rotation relative entre les deux parties de la gaine,
- la deuxième partie (16a) de la gaine dépasse du manchon (18), de façon à pouvoir être tournée par un utilisateur et détachée de la première partie (16b), la surface extérieure de l'embout (14) et la surface intérieure de la gaine étant séparées par un jeu, de façon que l'embout (14) ne risque pas de tourner solidairement à la deuxième partie (16a) de la gaine par frottement, et
- le dispositif comprend en outre des moyens (140, 164) de liaison en translation entre l'embout (14) et la deuxième partie (16a) de la gaine, configurés de manière que l'embout (14) et la deuxième partie (16a) de la gaine (16) peuvent être retirés solidairement de la seringue (1) sans faire tourner l'embout (14) autour de l'aiguille (10).

2. Dispositif selon la revendication 1, **caractérisé en ce que** les moyens de fixation comportent des pattes élastiques (166), conçues pour être encliquetées (F6, F7) élastiquement autour du nez (8) de la seringue (1).

3. Dispositif selon l'une des revendications précédentes, **caractérisé en ce que** les moyens de liaison en translation comportent des dents (164) ménagées sur une surface radiale interne de la gaine, qui coopèrent avec un épaulement radial (140) de l'embout.

4. Dispositif selon l'une des revendications précédentes, **caractérisé en ce que** le manchon (18) est opaque et délimite au moins un évidement (180) formant un guide pour un pion (160) porté par une première partie (16a) de la gaine (16).

5. Dispositif selon l'une des revendications précédentes, **caractérisé en ce que** les moyens de rappel du manchon comprennent un ressort hélicoïdal (20) ayant un sens d'enroulement à droite.

6. Seringue à aiguille collée (1), **caractérisée en ce qu'**elle comprend un dispositif de protection de l'aiguille (D) selon l'une des revendications précédentes.

7. Procédé de fabrication de seringues pré-remplies à aiguille collée, comprenant des étapes consistant à :
a) monter un dispositif (D) de protection de l'aiguille selon l'une des revendications précédentes sur des corps de seringue (2),
b) disposer les corps de seringue (2), équipés du dispositif de protection, dans des logements (O204) prévus dans un support (200),
c) placer le support (200) dans un container standard (100), fermer le container et stériliser le container pour le transport,
d) dans une ambiance stérile, sortir le support du container,
e) remplir chaque corps de seringue avec un principe actif (P),
**caractérisé en ce que** les dispositifs de protection (D) sont suffisamment compacts pour traverser les logements d'un support standard, lesquels ont un diamètre de 9,3 mm et **en ce que** le procédé comprend, en outre, les étapes suivantes :
f) insérer un piston (6) dans chaque seringue,
g) sortir les seringues (1) de leur support pour l'inspection, l'assemblage d'une tige de piston (4) standard et l'étiquetage, et à
h) emballer individuellement chaque seringue (1) avec un emballage primaire (300) et un emballage secondaire (400).

8. Procédé selon la revendication 7, **caractérisé en ce que**, à l'étape c), le support (200) est placé dans le container (100) de manière que les seringues (1) ne touchent pas le fond (102) du container (100).

9. Procédé selon la revendication 7 ou 8, **caractérisé en ce que** le dispositif (D) monté sur chaque corps de seringue présente à son extrémité distale un chanfrein annulaire (18.1) qui coopère sans accrocs avec un logement correspondant (O204) du support lors des étapes b) et g).

10. Procédé selon l'une des revendications 7 à 9 **caractérisé en ce que**, à l'étape g), la tige de piston (4) est vissée sur le piston (6).

11. Procédé selon l'une des revendications 7 à 10, **caractérisé en ce que** l'emballage primaire utilisé à l'étape g) est un emballage coque transparent (300).

12. Procédé selon l'une des revendications 7 à 11, **caractérisé en ce que** l'emballage secondaire utilisé à l'étape g) est une boite en carton (400).

13. Procédé selon l'une des revendications 7 à 12, **caractérisé en ce que** chaque dispositif comprend des moyens (166) de fixation élastique sur un corps de seringue correspondant, qui se fixent, lors de l'étape a), par rapprochement avec le corps de seringue.

14. Procédé selon la revendication 13, **caractérisé en ce que** les moyens de fixation incluent des pattes élastiques (166) qui coopèrent, lors de l'étape a), avec une partie d'extrémité (8) du corps de seringue, cette partie d'extrémité étant conformée pour bloquer le dégagement des pattes une fois le dispositif (D) monté.

15. Procédé selon l'une des revendications 7 à 14, **caractérisé en ce que** les étapes d) à f) sont automatisées.

## Patentansprüche

1. Vorrichtung (D) zum Schutz einer Nadel (10), die dafür vorgesehen ist, an einer Nase (8) eines Körper (2) einer Spritze (1) mit geklebter Nadel montiert zu werden, wobei diese Vorrichtung Folgendes umfasst:
- eine äußere Hülse (18), die entlang einer Längsachse (X1) zwischen einer vorgeschobenen Position, in der sie die Nadel bedeckt, und einer zurückgezogenen Position, in der sie die Nadel nicht bedeckt, beweglich ist,
- Mittel (20) zum Rückstellen der Hülse in die vorgeschobene Position, und
- Mittel (180d) zum Verriegeln der Hülse (18) in der vorgeschobenen Position am Ende der Injektion,
**dadurch gekennzeichnet, dass**:
- die Vorrichtung ferner einen starren Nadelschutz (12) umfasst, der einen biegsamen Ansatz (14) umfasst, der von einer starren Ummantelung (16) umhüllt ist, wobei diese Ummantelung einen ersten Teil (16b) und einen zweiten Teil (16a) umfasst, der von dem ersten Teil durch eine Drehbewegung in Bezug auf den ersten Teil abnehmbar ist, wobei der erste Teil (16b) der Ummantelung Mittel (166) zur elastischen Befestigung um die Nase (8) der Spritze umfasst, die es ermöglichen, den ersten Teil (16b) der Ummantelung und den Spritzenkörper (2) drehfest miteinander zu verbinden,
- die zwei Teile (16a, 16b) der Ummantelung durch teilbare Brücken (162) miteinander verbunden sind, die dafür ausgelegt sind, bei der Anwendung eines relativen Drehmoments (M1) zwischen den zwei Teilen der Ummantelung zerbrochen zu werden,
- der zweite Teil (16a) der Ummantelung von der Hülse (18) vorragt, um durch einen Benutzer gedreht und vom ersten Teil (16b) abgenommen werden zu können, wobei die Außenfläche des Ansatzes (14) und die Innenfläche der Ummantelung durch ein Spiel voneinander getrennt sind, sodass der Ansatz (14) keine Gefahr läuft, sich durch Reibung gemeinsam mit dem zweiten Teil (16a) der Ummantelung zu drehen, und
- die Vorrichtung ferner Mittel (140, 164) zur verschiebebeweglichen Verbindung zwischen dem Ansatz (14) und dem zweiten Teil (16a) der Ummantelung umfasst, die derart ausgebildet sind, dass der Ansatz (14) und der zweite Teil (16a) der Ummantelung (16) gemeinsam von der Spritze (1) gezogen werden können, ohne den Ansatz (14) um die Nadel (10) zu drehen.

2. Vorrichtung nach Anspruch 1, **dadurch gekennzeichnet, dass** die Befestigungsmittel elastische Klauen (166) umfassen, die dafür ausgelegt sind, um die Nase (8) der Spritze (1) elastisch einzurasten (F6, F7).

3. Vorrichtung nach einem der vorhergehenden Ansprüche, **dadurch gekennzeichnet, dass** die Mittel zur verschiebebeweglichen Verbindung Zähne (164) umfassen, die an einer radialen Innenfläche der Ummantelung gebildet sind und mit einer radialen Schulter (140) des Ansatzes zusammenwirken.

4. Vorrichtung nach einem der vorhergehenden Ansprüche, **dadurch gekennzeichnet, dass** die Hülse (18) undurchsichtig ist und wenigstens eine Aussparung (180) begrenzt, die eine Führung für einen Zapfen (160) bildet, der durch einen ersten Teil (16a) der Ummantelung (16) getragen wird.

5. Vorrichtung nach einem der vorhergehenden Ansprüche, **dadurch gekennzeichnet, dass** die Mittel zum Rückstellen der Hülse eine Spiralfeder (20) umfassen, die eine rechtsgängige Wickelrichtung aufweist.

6. Spritze mit geklebter Nadel (1), **dadurch gekennzeichnet, dass** sie eine Nadelschutzvorrichtung (D) nach einem der vorhergehenden Ansprüche umfasst.

7. Verfahren zur Herstellung von vorgefüllten Spritzen mit geklebter Nadel, umfassend Schritte, die in Folgendem bestehen:
a) Montieren einer Nadelschutzvorrichtung (D) nach einem der vorhergehenden Ansprüche an Spritzenkörpern (2),
b) Anordnen der mit der Schutzvorrichtung ausgestatteten Spritzenkörper (2) in Aufnahmen (O204), die in einer Halterung (200) vorgesehen sind,
c) Platzieren der Halterung (200) in einem Standardbehälter (100), Schließen des Behälters und Sterilisieren des Behälters zum Transport,
d) in einer sterilen Umgebung Herausnehmen der Halterung aus dem Behälter,
e) Füllen jedes Spritzenkörpers mit einem Wirkstoff (P),
**dadurch gekennzeichnet, dass** die Schutzvorrichtungen (D) ausreichend kompakt sind, um die Aufnahmen einer Standardhalterung zu durchdringen, die einen Durchmesser von 9,3 mm aufweisen, und dass das Verfahren ferner die folgenden Schritte umfasst:
f) Einfügen eines Kolbens (6) in jede Spritze,
g) Herausnehmen der Spritzen (1) aus ihrer Halterung zur Inspektion, zum Montieren einer Standard-Kolbenstange (4) und zur Etikettierung, und
h) einzelnes Verpacken jeder Spritze (1) mit einer primären Verpackung (300) und einer sekundären Verpackung (400).

8. Verfahren nach Anspruch 7, **dadurch gekennzeichnet, dass** im Schritt c) die Halterung (200) derart in dem Behälter (100) platziert wird, dass die Spritzen (1) den Boden (102) des Behälters (100) nicht berühren.

9. Verfahren nach Anspruch 7 oder 8, **dadurch gekennzeichnet, dass** die Vorrichtung (D), die an jedem Spritzenkörper montiert ist, an ihrem distalen Ende eine ringförmige Abschrägung (18.1) aufweist, die in den Schritten b) und g) reibungslos mit einer entsprechenden Aufnahme (O204) der Halterung zusammenwirkt.

10. Verfahren nach einem der Ansprüche 7 bis 9, **dadurch gekennzeichnet, dass** beim Schritt g) die Kolbenstange (4) auf den Kolben (6) geschraubt wird.

11. Verfahren nach einem der Ansprüche 7 bis 10, **dadurch gekennzeichnet, dass** die im Schritt g) verwendete primäre Verpackung eine transparente Blisterverpackung (300) ist.

12. Verfahren nach einem der Ansprüche 7 bis 11, **dadurch gekennzeichnet, dass** die im Schritt g) verwendete sekundäre Verpackung eine Kartonschachtel (400) ist.

13. Verfahren nach einem der Ansprüche 7 bis 12, **dadurch gekennzeichnet, dass** jede Vorrichtung Mittel (166) zum elastischen Befestigen an einem entsprechenden Spritzenkörper umfasst, die im Schritt a) durch Annähern an den Spritzenkörper befestigt werden.

14. Verfahren nach Anspruch 13, **dadurch gekennzeichnet, dass** die Befestigungsmittel elastische Klauen (166) beinhalten, die im Schritt a) mit einem Endteil (8) des Spritzenkörpers zusammenwirken, wobei dieser Endteil ausgebildet ist, um das Freigeben der Klauen zu blockieren, nachdem die Vorrichtung (D) montiert wurde.

15. Verfahren nach einem der Ansprüche 7 bis 14, **dadurch gekennzeichnet, dass** die Schritte d) bis f) automatisiert sind.

## Claims

1. A device (D) for protecting a needle (10), provided to be mounted on a nose (8) of a body (2) of a cemented needle syringe (1), this device comprising:
- an outer sleeve (18), which is movable along a longitudinal axis (X1) between a forward position, where it covers the needle, and a withdrawn position, where it does not cover the needle,
- return means (20) for returning the sleeve to the forward position, and
- means (180d) for locking the sleeve (18) in the forward position at the end of an injection
**characterized in that**:
- the device further includes a rigid needle shield (12) including a flexible end-piece (14) enclosed in a rigid sheath (16), this sheath comprising a first part (16a) and a second part (16b), which is detachable from the first part by a rotational movement relative to the first part, the first part (16b) of the sheath comprising fastening means (166) around the nose (8) of the syringe, which allow solidarization in rotation of the first part (16b) of the sheath with the body (2) of the syringe,
- the two parts (16a, 16b) of the sheath are connected to one another by sectile bridges (162), designed to be broken when relative torque (M1) is applied between the two parts of the sheath,
- the second part (16a) of the sheath protrudes out of the sleeve (18), so that it can be turned by a user and detached from the first part (16b), the outer surface of the end-piece (14) and the inner surface of the sheath being separated by a play, so that the end-piece (14) does not risk rotating jointly with the front part 16a of the sheath 16 by friction, and
- the device further includes means (140, 164) for translatable connection between the end-piece (14) and the second part (16a) of the sheath, configured such that the end-piece (14) and the second part (16a) of the sheath (16) can be removed jointly from the syringe (1) without rotating the end-piece (14) around the needle (10).

2. The device according to claim 1, **characterized in that** the fastening means include elastic tabs (166), designed to be elastically snapped (F6, F7) around the nose (8) of the syringe (1).

3. The device according to one of the preceding claims, **characterized in that** the translatable connecting means include teeth (164) arranged on an inner radial surface of the sheath, which cooperate with a radial shoulder (140) of the end-piece.

4. The device according to one of the preceding claims, **characterized in that** the sleeve (18) is opaque and defines at least one recess (180) forming a guide for a pin (160) supported by a first part (16a) of the sheath (16).

5. The device according to one of the preceding claims, **characterized in that** the return means of the sleeve comprise a helical spring (20) having a winding direction to the right.

6. A cemented needle syringe (1), **characterized in that** it comprises a device for protecting the needle (D) according to one of the preceding claims.

7. A method for manufacturing pre-filled cemented needle syringes, comprising steps consisting in:
a) mounting a device (D) for protecting the needle according to one of the preceding claims on syringe bodies (2),
b) positioning the syringe bodies (2), equipped with the protection device, in housings (O204) provided in a holder (200),
c) placing the holder (200) in a standard container (100), closing the container and sterilizing the container for transport,
d) in a sterile atmosphere, removing the holder from the container,
e) filling each syringe body with an active ingredient (P),
**characterized in that** the devices (D) for protecting are compact enough to go through the housings of a standard support, which have a diameter of 9.3 mm and **in that** the process further includes the following steps:
f) inserting a plunger (6) into each syringe,
g) removing the syringes (1) from their holder for inspection, assembling a plunger rod (4) and the label, and
h) individually packaging each syringe (1) with a primary packaging (300) and a secondary packaging (400).

8. The method according to claim 7, **characterized in that**, in step c), the holder (200) is placed in the container (100) such that the syringes (1) do not touch the bottom (102) of the container (100).

9. The method according to claim 7 or 8, **characterized in that** the device (D) mounted on each syringe body has, at its distal end, an annular bevel (18.1) that cooperates, without snags, with a corresponding housing (O204) of the holder during steps b) and g).

10. The method according to one of claims 7 to 9, **characterized in that**, in step g), the plunger rod (4) is screwed on the plunger (6).

11. The method according to one of claims 7 to 10, **characterized in that** the primary packaging used in step g) is a transparent shell packaging (300).

12. The method according to one of claims 7 to 11, **characterized in that** the secondary packaging used in step g) is a cardboard box (400).

13. The method according to one of claims 7 to 12, **characterized in that** each device comprises elastic fastening means (166) on a corresponding syringe body, which are fastened, during step a), by approaching the syringe body.

14. The method according to claim 13, **characterized in that** the fastening means include elastic tabs (166) that cooperate, during step a), with an end part (8) of the syringe body, this end part being configured to block the release of the tabs once the device (D) is mounted.

15. The method according to one of claims 7 to 14, **characterized in that** steps d) to f) are automatized.
